# EUROPÄISCHE PATENTANMELDUNG

(11) **EP 2 418 003 A2**
(43) Veröffentlichungstag der Anmeldung: **15.02.2012**
(21) Anmeldenummer: 11168048.4
(22) Anmeldetag: 30.05.2011
(51) Int. Cl.: A61Q 5/08, A61Q 5/10, A61K 8/22, A61K 8/25, A61K 8/49, A61K 8/92

(54) **Ammoniumsalz-haltige Blondiermittel**

(30) Priorität: 23.07.2010 DE 102010038316
(71) Anmelder: Henkel AG & Co. KGaA, 40589 Düsseldorf (DE)
(72) Erfinder: Günther, Katja, 40721 Hilden (DE); Janßen, Frank, 41470 Neuss (DE); Schmahl, Melanie, 40721 Hilden (DE)

(57) **Zusammenfassung**

Gegenstand der Erfindung ist ein zur Aufhellung und/oder Blondierung von keratinischen Fasern, welches unmittelbar vor der Anwendung auf der Faser aus mindestens 3 Teilen gemischt wird, wobei die 3 Teile
i) eine wasserfreie Zubereitung (A), enthaltend mindestens ein Peroxo-Salz,
ii) eine wässrige Zubereitung (B), enthaltend mindestens Wasserstoffperoxid, und
iii) eine Zubereitung (C), enthaltend in einem wässrigen kosmetischen Träger mindestens eine Ammonium-Verbindung der Formel (I),

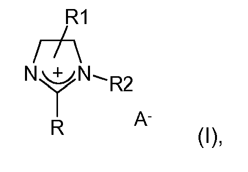

umfassen.

## Beschreibung

Die vorliegende Erfindung betrifft Blondiermittel zum Aufhellen von keratinischen Fasern, insbesondere menschlicher Haare. Weiterhin die vorliegende Erfindung betrifft Verwendung der Mittel zur schonenden Blondierung von menschlichen Haaren sowie eine Mehrkomponentenverpackungseinheit (Kit-of-Parts) zur Aufhellung keratinischer Fasern, welche getrennt voneinander konfektioniert eine Zubereitung mit einer bestimmten Ammoniumverbindung, eine Oxidationsmittelzubereitung und ein Blondierpulver umfasst.

Das Aufhellen der eigenen Haarfarbe ist seit jeher der Wunsch vieler Verbraucher, da eine blonde Haarfarbe als attraktiv und in modischer Hinsicht erstrebenswert betrachtet wird. Dazu sind im Markt verschiedene Blondiermittel mit unterschiedlicher Blondierleistung erhältlich. Die in diesen Produkten enthaltenen Oxidationsmittel sind in der Lage, durch die oxidative Zerstörung des haareigenen Farbstoffes Melanin und/oder künstlicher Farbstoffe die Haarfaser aufzuhellen. Für einen moderaten Blondiereffekt genügt der Einsatz von Wasserstoffperoxid - gegebenenfalls unter Einsatz von Ammoniak oder anderen Alkalisierungsmitteln - als Oxidationsmittel allein, für das Erzielen eines stärkeren Blondiereffektes wird üblicherweise eine Mischung aus Wasserstoffperoxid und Peroxo-Salzen, insbesondere Peroxodisulfatsalzen, eingesetzt.

Diese Peroxo-Salze werden üblicherweise in Form eines Pulvers der Wasserstoffperoxid-Zubereitung unmittelbar vor der Anwendung zugegeben, um Instabilitäten bei der Lagerung und Schwankungen beim Wasserstoffperoxidgehalt zum Zeitpunkt der Anwendung zu vermeiden.

Mit einer verstärkten Aufhellung geht jedoch auch eine Schädigung des Haares einher, da nicht nur die Farbstoffe des Haares, sondern auch die übrigen Strukturbestandteile des Haares oxidativ geschädigt werden. Je nach Ausprägung des Schädigungsgrades reicht dieser von rauem, sprödem und schwieriger auskämmbarem Haar über eine verminderte Widerstandsfähigkeit und Reißfestigkeit des Haares sowie Spliss bis hin zu Haarbruch. Je länger die Einwirkzeit und je größer die Menge des eingesetzten Wasserstoffperoxids und der Peroxodisulfate ist, desto stärkere Schädigungen werden in der Regel auf der Keratinfaser hervorgerufen. Zur Verbesserung des Pflegezustands der Haare werden Haarbehandlungsmitteln üblicherweise Pflegestoffe zugesetzt. Die drastischen Bedingungen, insbesondere alkalischer pH-Wert und Zusatz von Oxidationsmitteln, bei einer oxidativen Haaraufhellung führen jedoch dazu, dass übliche Pflegestoffe die gewünschte Pflegeleistung aufgrund mangelnder Stabilität nicht ausreichend erbringen können. Die Pflegeleistung lässt sich in vielfältiger Weise bestimmen. Dazu gehören Methoden, die auf die Schädigung der inneren Struktur der Haare gerichtet sind, wie NIR-Bestimmungen des Cysteinsäureanteils im Keratin oder DSC-Schmelzpunkt-Messungen, ebenso wie Methoden, die auf die Haaroberfläche abzielen, wie Kämmbarkeit. Außerdem kann die Bestimmung von Spliss oder Haarbruch als Maß für eine Pflegeleistung der Mittel dienen.

Aufgabe dieser Erfindungsmeldung ist es daher, neuartige Mittel zum Aufhellen bzw. Blondieren von Haaren, insbesondere menschlichen Haaren bereitzustellen, welche neben einer guten Aufhellleistung eine verringerte Haarschädigung und insgesamt ansprechende Pflegeleistung aufweisen und den Haaren eine verbesserte Kämmbarkeit verleihen.

Aus US2007/0104671 A1 sind Konditioniermittel mit bestimmten kationischen Verbindungen, darunter auch kationische Imidazoliniumverbindungen, bekannt. EP1308151 A1 offenbart wasserfreie Blondiermittelsuspensionen mit lipophil modifizierten Tonmineralen, wie Quaternium-18 Bentonit, als Verdickungsmittel. Keines der Dokumente gibt jedoch irgendeinen Hinweis auf verbesserte Pflege oder verringerte Schädigung bei der oxidativen Faserbehandlung.

Im Zuge der Untersuchungen der Anmelderin stellte sich jedoch unerwartet heraus, dass sich die erfindungsgemäße Aufgabe durch die Einarbeitung bestimmter Ammonium-Verbindungen in eine Zubereitung eines mehrteiligen Blondiermittels gelöst wird.

Ein erster Gegenstand der vorliegenden Erfindung ist daher ein Mittel zur Aufhellung und/oder Blondierung von keratinischen Fasern, welches unmittelbar vor der Anwendung auf der Faser aus mindestens 3 Teilen gemischt wird, und welches dadurch gekennzeichnet ist, dass die 3 Teile
i) eine wasserfreie Zubereitung (A), enthaltend mindestens ein Peroxo-Salz,
ii) eine wässrige Zubereitung (B), enthaltend mindestens Wasserstoffperoxid, und
iii) eine Zubereitung (C), enthaltend in einem wässrigen kosmetischen Träger mindestens eine Ammonium-Verbindung der Formel (I),

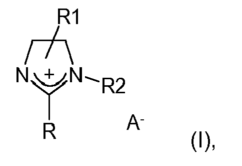

worin
- R: für eine gesättigte oder ungesättigte, lineare oder verzweigte C₇-C₂₉-Kohlenwasserstoffgruppe,
- R1: für eine Cₗ-C₆-Alkylgruppe, eine C₂-C₆-Alkenylgruppe oder eine C₂-C₆-Hydroxyalkylgruppe,
- R2: für (CH₂)ₙCO₂H mit n als ganzer Zahl von 1 bis 5, oder für (CH₂)ₘXC(=O)R', worin m für eine ganze Zahl von 1 bis 5, X für O oder NH und R' für eine gesättigte oder ungesättigte, lineare oder verzweigte C₇-C₂₉-Kohlenwasserstoffgruppe stehen, und
- A: für ein physiologisch verträgliches Anion stehen,
umfassen.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Die wasserfreien Zubereitungen (A) sind bevorzugt pulverförmig. Unter "pulverförmigen" Mitteln sind im Rahmen der vorliegenden Erfindung solche Mittel zu verstehen, die aus zerkleinerten, festen Bestandteilen bestehen, wobei die Zerkleinerung durch Zerreiben, Zerstoßen, Mahlen oder durch Zerstäubungstrocknungen oder Gefriertrocknungen erzielt werden kann. Dabei können Pulver aus festen Bestandteilen mit unterschiedlichen Korngrößen eingesetzt werden. Üblicherweise kann es bevorzugt sein, wenn die Pulver jedoch eine möglichst homogene Korngröße aufweisen, insbesondere um eine einheitliche Dispersion bzw. Auflösung der Pulver in den Zubereitungen (B) und (C) zu erleichtern.

Die Zubereitungen (A) enthalten die Wirkstoffe in einem festen kosmetischen Träger. Dieser feste kosmetische Träger kann insbesondere Salze der Kieselsäure, insbesondere von Ammonium, Alkalimetallen sowie Erdalkalimetallen der Silicate und Metasilicate. Insbesondere Metasilicate, die sich gemäß Formel (SiO₂)ₙ(M₂O)ₘ , wobei M für ein Ammoniumion, ein Alkalimetall oder ein halbes Stöchiometrieäquivalents eines Erdalkalimetalls steht, durch das Verhältnis zwischen n und m von ≤ 1 auszeichnen und sich als kettenförmige polymere Strukturen des Anions [SiO₃]²⁻ auffassen lassen, können bevorzugt eingesetzt werden. Natriummetasilicat der Formel [Na₂SiO₃]_{∞}, ist dabei besonders bevorzugt. Erfindungsgemäß gleichfalls bevorzugt sind solche Silicate, die aus einem Silicat der Formel (SiO₂)ₙ(Na₂O)m(K₂O)ₚ gebildet werden, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 2:1 und 4:1 liegt.

Weiterhin können die festen kosmetischen Träger sogenannte Rieselhilfen enthalten, die ein Verklumpen oder Verbacken der Pulver-Bestandteile verhindern sollen. Als solche Rieselhilfen kommen bevorzugt wasserunlösliche, hydrophobierende oder Feuchtigkeit adsorbierende Pulver von Kieselgur, pyrogenen Kieselsäuren, Calciumphosphat, Calciumsilicaten, Aluminiumoxid, Magnesiumoxid, Magnesiumcarbonat, Zinkoxid, Stearaten, Fettaminen und dergleichen in Frage.

Schließlich können die festen kosmetischen Träger noch zusätzlich ein Entstaubungsmittel enthalten, die die Staubbildung der pulverförmigen Bestandteile verhindert. Hierzu können insbesondere inerte Öle eingesetzt werden. Bevorzugt enthalten die festen, kosmetischen Träger als Entstaubungsmittel Esteröle oder Mineralöle, bevorzugt Kohlenwasseröle, wie flüssiges Paraffinöl.

Als ersten wesentlichen Inhaltsstoff enthält die Zubereitung (A) des ersten Erfindungsgegenstands mindestens ein Peroxo-Salz. Erfindungsgemäß geeignete Peroxo-Salze sind anorganische Peroxoverbindungen. Diese sind bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate.

Eine Ausführungsform der vorliegenden Erfindung ist dadurch gekennzeichnet, dass die Zubereitung (A) als Peroxo-Salz mindestens ein Peroxodisulfat-Salz, insbesondere ausgewählt aus der Gruppe, gebildet aus Ammoniumperoxodisulfat, Kaliumperoxodisulfat und Natriumperoxodisulfat, enthält.

Weiterhin hat es sich bei den Arbeiten zur vorliegenden Erfindung als besonders bevorzugt erwiesen, wenn die Zubereitung (A) des ersten Erfindungsgegenstands mindestens zwei verschiedene Peroxodisulfate enthalten. Bevorzugte Peroxodisulfat-Salze sind dabei Kombinationen aus Ammoniumperoxodisulfat mit Kaliumperoxodisulfat und/oder Natriumperoxodisulfat.

Die Zubereitungen (A) enthalten Peroxo-Salze in einer Menge von 0,1 bis 80 Gew.-%, bevorzugt von 1 bis 60 Gew.-% und besonders bevorzugt von 2 bis 50 Gew.-%, jeweils bezogen auf das Gesamtgewicht des Mittels.

Die Zubereitung (A) kann zur Verstärkung der Blondierwirkung noch zusätzliche Bleichkraftverstärker enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. i-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker vom Typ der Kohlensäurederivate können erfindungsgemäß bevorzugt Carbonatsalze bzw. Hydrogencarbonatsalze eingesetzt werden. Diese sind bevorzugt ausgewählt aus der Gruppe der Ammonium-, Alkalimetall- (insbesondere Na und K) sowie Erdalkalimetall-(insbesondere Mg und Ca), -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Natriumcarbonat, Kaliumhydrogencarbonat, Kaliumcarbonat, Magnesiumcarbonat und Calciumcarbonat. Diese besonders bevorzugten Salze können allein oder in deren Mischungen von mindestens zwei Vertretern als Bleichverstärker verwendet werden.

Weiterhin sind Alkylcarbonate, -carbamate, Silylcarbonate sowie -carbamate geeignete Bleichverstärker.

Weiterhin erfindungsgemäß einsetzbare Bleichkraftverstärker können aus stickstoffhaltigen, gegebenenfalls kationischen Heterocyclen, insbesondere Imidazol, ausgewählt werden.

Besonders bevorzugte stickstoffhaltige, heterocyclische Bleichkraftverstärker sind die quaternierten Kationen von Pyridinen und 3,4-Dihydroisochinolinen, wie Salze des 4-Acetyl-1-methylpyridiniums, insbesondere 4-Acetyl-1-methylpyridinium-p-toluolsulfonat, Salze des 2-Acetyl-1-methylpyridiniums, insbesondere 2-Acetyl-1-methylpyridinium-p-toluolsulfonat, sowie Salze des N-Methyl-3,4-dihydroisochinoliniums, insbesondere N-Methyl-3,4-dihydroisochinolinium-p-toluolsulfonat.

Die neben den Peroxo-Salzen eingesetzten Bleichkraftverstärker sind in der Zubereitung (A) bevorzugt in Mengen von 0,5 bis 30 Gew.-%, insbesondere in Mengen von 2 bis 20 Gew.-%, jeweils bezogen auf das Gesamtgewicht der Blondierzubereitung (B), enthalten.

Zur weiteren Steigerung der Aufhellleistung kann der erfindungsgemäßen Zubereitung (A) des ersten Erfindungsgegenstands zusätzlich als Bleichverstärker eine Silicium-haltige Verbindung zugesetzt werden. Diese wird bevorzugt ausgewählt aus der Gruppe, die gebildet wird aus Kieselsäure, Alkalimetallsilicaten und Erdalkalimetallsilicaten.

Eine weitere Ausführungsform des ersten Erfindungsgegenstands ist daher dadurch gekennzeichnet, dass die Zubereitung (A) zusätzlich mindestens eine Silicium-haltige Verbindung enthält, die ausgewählt ist aus der Gruppe, die gebildet wird aus Kieselsäure, Alkalimetallsilicaten und Erdalkalimetallsilicaten.

Obwohl bereits geringe Mengen der Silicium-haltigen Verbindungen die Aufhellleistung erhöhen, kann es erfindungsgemäß bevorzugt sein, die Silicium-haltigen Verbindungen in Mengen von 0,05 Gew.-% bis 50 Gew.-%, bevorzugt in Mengen von 0,5 Gew.-% bis 30 Gew.-% und besonders bevorzugt in Mengen von 1,0 Gew.-% bis 25 Gew.-%, jeweils bezogen auf das Gesamtgewicht der Zubereitung (A), einzusetzen.

Als Silicium-haltige Verbindungen werden insbesondere Alkalimetallsilicate in Form von Wasserglas eingesetzt. Unter Wasserglas ist dabei eine Verbindung zu verstehen, die aus einem Silicat der Formel (SiO₂)ₙ(Na₂O)ₘ(K₂O)ₚ gebildet wird, wobei n für eine positive rationale Zahl und m und p unabhängig voneinander für eine positive rationale Zahl oder für 0 stehen, mit den Maßgaben, dass mindestens einer der Parameter m oder p von 0 verschieden ist und das Verhältnis zwischen n und der Summe aus m und p zwischen 1:1 und 4:1 liegt.

Neben den durch die Summenformel beschriebenen Komponenten können die Wassergläser in geringen Mengen noch weitere Zusatzstoffe, wie Phosphate oder Magnesiumsalze, enthalten.

Erfindungsgemäß besonders bevorzugte Wassergläser werden unter anderem unter den Bezeichnungen Ferrosil^{®} 119, Natronwasserglas 40/42, Portil^{®} A, Portil^{®} AW und Portil^{®} W und Britesil^{®} C20 vertrieben.

Weiterhin werden als Silicium-haltige Verbindungen insbesondere Kieselsäuren eingesetzt, die auch als Silica oder Kieselgel vertrieben werden. Bevorzugt ist dabei ein Kieselgel, welches unter dem Handelsnamen Aerosil 200 (lNCl-Bezeichnung: Silica) vertrieben wird.

Als wesentlichen Inhaltstoff enthält die Zubereitung (C) des Blondiermittels des ersten Erfindungsgegenstands mindestens eine Ammonium-Verbindung der Formel (I),

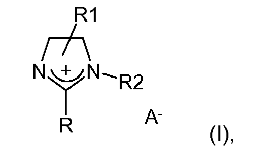

worin
- R: für eine gesättigte oder ungesättigte, lineare oder verzweigte C₇-C₂₉-Kohlenwasserstoffgruppe,
- R1: an ein Stickstoffatom im Imidazolin-Ring gebunden ist und für eine C₁-C₆-Alkylgruppe, eine C₂-C₆-Alkenylgruppe oder eine C₂-C₆-Hydroxyalkylgruppe,
- R2: für (CH₂)ₘXC(=O)R', worin m für eine ganze Zahl von 1 bis 5, X für O oder NH und R' für eine gesättigte oder ungesättigte, lineare oder verzweigte C₇-C₂₉Kohlenwasserstoffgruppe stehen, oder für (CH₂)ₙCO₂H mit n als ganzer Zahl von 1 bis 5, und
- A⁻: für ein physiologisch verträgliches Anion stehen,
umfassen.

Die Reste R sowie gegebenenfalls R' der Formel (I) stehen dabei unabhängig voneinander für eine gesättigte oder ungesättigte, lineare oder verzweigte C₇-C₂₉-Kohlenwasserstoffgruppe. Bevorzugt leiten sich diese Kohlenwasserstoffe von Fettresten ab, welche gegebenenfalls gesättigte und/oder ungesättigte Strukturelemente sowie gegebenenfalls Verzweigungen aufweisen. Dies wird im Namen des Fettrests durch das Präfix "Nor" ausgedrückt, was für einen entsprechenden, um ein C-Atom kettenverkürzten Rest steht. Bevorzugt sind dabei insbesondere ein Nor-Decyl- (C9:0), ein Nor-Lauryl- (C11:0), ein Nor-Myristyl- (C13:0), ein Nor-Cetyl- (C15:0), ein Nor-Stearyl- (17:0) oder ein Nor-Oleyl-Rest (C17:1). Die Angaben in Klammer beziehen sich dabei Kohlenstoffanzahl der Fettreste sowie nachgestellt auf die Anzahl der enthaltenen Ungesättigtheiten in Form von C-C-Doppelbindungen. Je nach Herkunft und Herstellmethode können die Reste R und R' auch jeweils unabhängig voneinander als ein Gemisch von gesättigten oder ungesättigten, linearen oder verzweigten C₇-C₂₉-Kohlenwasserstoffgruppen mit einer unterschiedlichen Kohlenstoffanzahl in der Gruppe vorliegen. Solche Gemische werden unter Oberbegriffen zusammengefasst. Bevorzugt sind hierbei ein Nor-Cetearyl- (überwiegend C15:0 und C17:0), ein Nor-Cocoalkyl- (überwiegend C11:0, C13:0 und C15:0), ein Nor-Palmalkyl (überwiegend gesättigte und ungesättigte C9, C11, C13, C15, C17 und C19), ein Nor-Talgalkyl- (überwiegend gesättigte und ungesättigte C13, C15 und C17) sowie ein hydrierter Nor-Talgalkyl-Rest (überwiegend C13:0, C15:0 und C17:0).

Besonders bevorzugt stehen die Reste R und gegebenenfalls R' jeweils unabhängig voneinander für eine Nor-Decyl-, eine Nor-Lauryl-, eine Nor-Myristyl-, eine Nor-Cetyl-, eine Nor-Cetearyl-, eine Nor-Cocoalkyl-, eine Nor-Palmalkyl-, eine Nor-Talgalkyl-, eine hydrierte Nor-Talgalkyl-, eine Nor-Stearyl- und/oder eine Nor-Oleyl-Gruppe.

Der Rest R1 ist dabei an eines der beiden Stickstoffatome des Imidazolinrings gebunden und steht für eine C₁-C₆-Alkylgruppe, eine C₂-C₆-Alkenylgruppe oder eine C₂-C₆-Hydroxyalkylgruppe. Bevorzugt sind dabei eine Methyl-, eine Ethyl-, eine 2-Methylethyl-, eine Allyl- und/oder eine 2-Hydroxyethyl-Gruppe. Besonders bevorzugt steht R1 für eine Methyl-Gruppe und/oder eine 2-Hydroxyethyl-Gruppe.

Der Rest R2 in Formel (I) steht für eine Gruppe (CH₂)ₘXC(=O)R', worin m für eine ganze Zahl von 1 bis 5, X für O oder NH und R' für eine gesättigte oder ungesättigte, lineare oder verzweigte C₇-C₂₉-Kohlenwasserstoffgruppe stehen, oder für eine Gruppe (CH₂)ₙCO₂H, wobei n für eine ganze Zahl von 1 bis 5 steht.

Als physiologisch verträgliches Anion A- eignen sich erfindungsgemäß alle kosmetisch einsetzbaren Anionen. Bevorzugt sind Halogenide, insbesondere Chlorid, sowie Sulfonate, wie Toluolsulfonat, Methylsulfonat oder Phenylsulfonat.

In einer Ausführungsform steht der Rest R2 in Formel (I) eine Gruppe (CH₂)ₘXC(=O)R', worin m für eine ganze Zahl von 1 bis 5, X für O oder NH und R' für eine gesättigte oder ungesättigte, lineare oder verzweigte C₇-C₂₉-Kohlenwasserstoffgruppe stehen.

Bevorzugt ist dabei, dass X für NH steht. Bevorzugt steht m für eine Zahl 2 oder 3, bevorzugt für 2, wobei es besonders bevorzugt ist, wenn X für NH und m gleichzeitig für die Zahl 2 stehen.

Bevorzugte Verbindungen der Formel (I), in der der Rest R2 in Formel (I) für eine Gruppe (CH₂)ₘXC(=O)R' stehen, sind die unter den INCI-Bezeichnungen Quaternium-87 (4,5-Dihydro-1-Methyl-2-Nor-Palmalkyl-1-(2-Nor-Palmalkylamido)ethyl Imidazolium Methylsulfat) und Quaternium-27 (4,5-Dihydro-1-Methyl-2-Nor-Talgalkyl-1-(2-Nor-Talgalkylamido)ethyl Imidazolium Methylsulfat ) bekannte Verbindungen.

In einer weiteren Ausführungsform steht der Rest R2 in Formel (I) für eine Gruppe (CH₂)ₙCO₂H. Wenn der Rest R2 für eine Gruppe (CH₂)ₙCO₂H steht, kann die Carboxygruppe auch deprotoniert vorliegen, so dass sich mit der positiven im Imidazolinium-Ring eine betainische Struktur ausbildet. Bevorzugt steht n für eine Zahl 1 oder 2, bevorzugt für 1.

Besonders bevorzugte Verbindungen der Formel (I) sind dadurch gekennzeichnet, dass der Rest R1 für eine 2-Hydroethyl-Gruppe und der Rest R2 eine Gruppe (CH₂)ₙCO₂H steht, wobei n für die Zahl 1 steht, wobei die Carboxygruppe insbesondere deprotoniert vorliegt.

Eine bevorzugte Verbindung der Formel (I), in der der Rest R2 in Formel (I) für eine Gruppe (CH₂)ₙCO₂H steht, ist 1-Carboxymethyl-4,5-dihydro-1-(2-hydroxyethyl)-2-nor-cocoalkyl-imidazolium, inneres Salz, und wird unter der INCI Bezeichnung Natrium Cocoamphoacetate vertrieben.

Die Ammonium-Verbindungen der Formel (I) sind im erfindungsgemäßen Mittel bevorzugt in einer Menge von 0,01 bis 20 Gew.-%, insbesondere von 0,5 bis 5 Gew.-% und besonders bevorzugt von 0,1 bis 3 Gew.-%, jeweils bezogen das Gesamtgewicht des Mittels, enthalten.

Eine weitere bevorzugte Ausführungsform des ersten Erfindungsgegenstands ist daher ein Mittel zur Aufhellung und/oder Blondierung von keratinischen Fasern, welches unmittelbar vor der Anwendung auf der Faser aus mindestens 3 Teilen gemischt wird, und welches dadurch gekennzeichnet ist, dass die 3 Teile
i) eine wasserfreie Zubereitung (A), enthaltend mindestens ein Peroxodisulfat-Salz, insbesondere ausgewählt aus der Gruppe, gebildet aus Ammoniumperoxodisulfat, Kaliumperoxodisulfat und Natriumperoxodisulfat,
ii) eine wässrige Zubereitung (B), enthaltend mindestens Wasserstoffperoxid, und
iii) eine Zubereitung (C), enthaltend in einem wässrigen kosmetischen Träger mindestens Quaternium-87,
umfassen.

Eine weitere bevorzugte Ausführungsform des ersten Erfindungsgegenstands ist daher ein Mittel zur Aufhellung und/oder Blondierung von keratinischen Fasern, welches unmittelbar vor der Anwendung auf der Faser aus mindestens 3 Teilen gemischt wird, und welches dadurch gekennzeichnet ist, dass die 3 Teile
i) eine wasserfreie Zubereitung (A), enthaltend mindestens ein Peroxodisulfat-Salz, insbesondere ausgewählt aus der Gruppe, gebildet aus Ammoniumperoxodisulfat, Kaliumperoxodisulfat und Natriumperoxodisulfat,
ii) eine wässrige Zubereitung (B), enthaltend mindestens Wasserstoffperoxid, und
iii) eine Zubereitung (C), enthaltend in einem wässrigen kosmetischen Träger mindestens Quaternium-27,
umfassen.

Eine weitere bevorzugte Ausführungsform des ersten Erfindungsgegenstands ist daher ein Mittel zur Aufhellung und/oder Blondierung von keratinischen Fasern, welches unmittelbar vor der Anwendung auf der Faser aus mindestens 3 Teilen gemischt wird, und welches dadurch gekennzeichnet ist, dass die 3 Teile
i) eine wasserfreie Zubereitung (A), enthaltend mindestens ein Peroxodisulfat-Salz, insbesondere ausgewählt aus der Gruppe, gebildet aus Ammoniumperoxodisulfat, Kaliumperoxodisulfat und Natriumperoxodisulfat,
ii) eine wässrige Zubereitung (B), enthaltend mindestens Wasserstoffperoxid, und
iii) eine Zubereitung (C), enthaltend in einem wässrigen kosmetischen Träger mindestens Natrium Cocoamphoacetate,
umfassen.

Die Zubereitung (B) sowie die Zubereitung (C) enthalten die Wirkstoffe in einem wässrigen, kosmetischen Träger. Der wässrige, kosmetische Träger ist dabei bevorzugt wässrig oder wässrigalkoholisch. Zum Zwecke der Haarbleiche sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Ein wässriger Träger enthält im Sinne der Erfindung mindestens 40 Gew.-%, insbesondere mindestens 50 Gew.-% Wasser. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether enthalten. Bevorzugt sind dabei alle wasserlöslichen organischen Lösungsmittel.

Die Zubereitung (B) enthält als wesentlichen Inhaltsstoff Wasserstoffperoxid. In einer bevorzugten Ausführungsform wird Wasserstoffperoxid selbst als wässrige Lösung verwendet.

Die Konzentration einer Wasserstoffperoxid-Lösung in der Oxidationsmittelzubereitung (B) wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie, bezogen auf das Gesamtgewicht des anwendungsbereiten Mittels, 0,01 bis 12 Gew.-%, vorzugsweise 0,1 bis 10 Gew.-%, besonders bevorzugt 1 bis 6 Gew.-% Wasserstoffperoxid enthalten.

Die Zubereitung (C) kann in einer weiteren Ausführungsform mindestens ein Alkalisierungsmittel enthalten. Die erfindungsgemäß verwendbaren Alkalisierungsmittel werden bevorzugt aus der Gruppe, gebildet aus Ammoniak, Alkanolaminen, basischen Aminosäuren,sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt. Als Metallionen dienen bevorzugt Lithium, Natrium und/oder Kalium.

Erfindungsgemäß einsetzbare Alkalisierungsmittel werden bevorzugt ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Methylglucamin, Triethanolamin, Diethanolamin und Triisopropanolamin. Insbesondere bevorzugte Alkanolamine sind Monoethanolamin, 2-Amino-2-methylpropanol und Triethanolamin.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D/L-Arginin, L-Lysin, D-Lysin, D/L-Lysin, L-Ornithin, D- Ornithin, D/L- Ornithin, L-Histidin, D-Histidin und D/L-Histidin, besonders bevorzugt L-Arginin, D-Arginin, D/L-Arginin als ein Alkalisierungsmittel im Sinne der Erfindung eingesetzt.

Bevorzugt sind Alkalisierungsmittel in einer Menge von 0,05 bis 20 Gew.-%, insbesondere von 0,5 bis 10 Gew.-%, jeweils bezogen auf das Gesamtgewicht des anwendungsbereiten Mittels, enthalten.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 7 und 12, insbesondere zwischen 8 und 11, liegt. Bei den pH-Werten im Sinne der vorliegenden Erfindung handelt es sich um pH-Werte, die bei einer Temperatur von 22 °C gemessen wurden.

Die Zubereitung (C) kann in einer weiteren Ausführungsform mindestens ein pflanzliches Öl zur weiteren Verbesserung der Pfelgeleistung auf dem Haar enthalten. Besonders geeignet sind dabei pflanzliche Öle auf Triglycerid-Basis, die einen hohen Anteil an ungesättigten Fettsäurebestandteilen enthalten. Bevorzugte ungesättigte Fettsäureanteile sind insbesondere solche auf Basis von Ölsäure, Palmitoleylsäure, Linolsäure, Linolensäure und Arachidonsäure, insbesondere Linolsäure. Beispielhafte pflanzliche Öle mit einem hohen Anteil an Triglyceridbestandteilen auf Basis von Linolsäure sind Sonnenblumenöl, Sojabohnenöl, Leinöl, Baumwollsaatöl, Rapsöl, Maiskeimöl, Weizenkeimöl und Erdnussöl.

Eine weitere bevorzugte Ausführungsform des ersten Erfindungsgegenstands ist daher ein Mittel, welches dadurch gekennzeichnet ist, dass die Zubereitung (C) zusätzlich mindestens ein pflanzliches Öl, ausgewählt aus der Gruppe, die gebildet wird aus Sonnenblumenöl, Sojabohnenöl, Leinöl, Baumwollsaatöl, Rapsöl, Maiskeimöl, Weizenkeimöl und Erdnussöl, enthält.

Sonnenblumenöl ist dabei ein besonders bevorzugtes pflanzliches Öl.

Bevorzugt sind die pflanzlichen Öle im anwendungsbereiten Mittel in einer Menge von 0,05 bis 20 Gew.-%, insbesondere von 0,1 bis 10 Gew.-%, jeweils bezogen auf das Gesamtgewicht des anwendungsbereiten Mittels, enthalten.

Erfindungsgemäß kann das anwendungsbereite Mittel auch zusammen mit einem Katalysator auf das Haar aufgebracht werden. Solche Katalysatoren sind z. B. bestimmte Enzyme, insbesondere Peroxidasen, lodide, Chinone oder Metallionen, wie beispielsweise Zn ²⁺, Cu²⁺, Fe²⁺ , Fe³⁺, Mn²⁺, Mn⁴⁺ , Li⁺, Mg²⁺, Ca²⁺, Ce⁴⁺, V³⁺, Co²⁺, Ru³⁺ und Al³⁺, insbesondere Zn ²⁺, Cu²⁺ und Mn ²⁺.

Weiterhin hat es sich als vorteilhaft erweisen, wenn die anwendungsbereiten Mittel mindestens einen Stabilisator oder Komplexbildner enthalten, die in einer oder mehrerer der Zubereitungen (A), (B) und (C) vorlegt werden. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Diese können unterschiedlichen chemischen Gruppen angehören. Vorzugsweise werden einzeln oder im Gemisch miteinander eingesetzt. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Die anwendungsbereiten Mittel können weiterhin zusätzliche Wirk-, Hilfs- und Zusatzstoffe enthalten, wobei die Wirkstoffe je nach Kompatibilität in einer oder mehrer der Zubereitungen (A), (B) und (C) vorlegt werden.

Vorzugsweise werden die anwendungsbereiten Mittel zur Aufhellung und/oder Blondierung als fließfähigen Zubereitung bereitgestellt und daher zusätzlich ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Mengen von 0,1 1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Besonders geeignete zwitterionische Tenside sind die sogenannten Betaine und N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist das unter der INCI-Bezeichnung Cocamidopropyl Betaine bekannte Fettsäureamid-Derivat.

Erfindungsgemäß bevorzugte Mittel sind dadurch gekennzeichnet, dass das Mittel zusätzlich mindestens ein amphoteres Tensid enthält. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind das N-Kokosalkylaminopropionat, das Kokosacylaminoethylaminopropionat und das C₁₂-C₁₈-Acylsarcosin. Weiterhin hat es sich als vorteilhaft erwiesen, wenn die Mittel weitere, nichtionogene grenzflächenaktive Stoffe, enthalten. Als bevorzugte nichtionische Tenside haben sich Alkylpolyglykoside sowie Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können weitere kationische Tenside vom Typ der quartären Ammoniumverbindungen, die nicht der Formel (I) aus Anspruch 1 entsprechen, der Esterquats und der Amidoamine enthalten. Bevorzugte quaternäre Ammoniumverbindungen sind Trialkylmethylammoniumhalogenide, wie Cetrimoniumchlorid oder Dialkyldimethylammoniumhalogenide. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid^{®} S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Die anwendungsbereiten Aufhellmittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind
- anionische, synthetische Polymere;
- kationische, synthetische Polymere;
- natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen;
- nichtionische, vollsynthetische Polymere, wie Polyvinylalkohol oder Polyvinylpyrrolidinon; sowie
- anorganische Verdickungsmittel, insbesondere Schichtsilikate wie beispielsweise Bentonit, besonders Smektite, wie Montmorillonit oder Hectorit.

Weiterhin können die Aufhell- bzw. Blondiermittel zur Mattierung von unerwünschten Restfarbeindrücken, insbesondere im rötlichen oder bläulichen Bereich, bestimmte, direktziehende Farbstoffe der komplementären Farben enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe sind als anionische, kationische und nichtionische direktziehende Farbstoffe bekannt. Die direktziehenden Farbstoffe werden jeweils bevorzugt in einer Menge von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen. Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe. Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Ganz besonders bevorzugt sind Mittel, die mindestens eine Kombination aus Tetrabromphenolblau und Acid Red 92 enthalten.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxyund/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazoliniummethochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide, insbesondere Arginin und/oder Serin; Proteinhydrolysate auf tierischer und/oder pflanzlicher Basis, wie beispielsweise Elastin-, Kollagen-, Keratin-, Seiden- und Milcheiweiß-Proteinhydrolysate, oder Mandel-, Reis-, Erbsen-, Kartoffelund Weizenproteinhydrolysate, sowie in Form ihrer Fettsäure-Kondensationsprodukte oder gegebenenfalls anionisch oder kationisch modifizierten Derivate; pflanzliche Öle, wie Macadamianussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl; Lichtschutzmittel, wie derivatisierte Benzophenone, Zimtsäure-Derivate und Triazine; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen, insbesondere der Gruppen A, B₃, B₅, B₆, C, E, F und H; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Die anwendungsbereiten Mittel des ersten Erfindungsgegenstands werden unmittelbar vor der Anwendung auf dem Haar durch Mischen der 3 Zubereitungen (A), (B) und (C) hergestellt. Dabei kann es unerheblich sein, ob zunächst 2 Zubereitungen miteinander vermischt werden und anschließend die dritte Zubereitung zugegeben und untergemischt wird, oder ob alle 3 Zubereitungen gemeinsam zusammengeführt werden und anschließend vermischt werden. Das Vermischen kann durch Verrühren in einer Schale oder einem Becher erfolgen oder durch Schütteln in einem verschließbaren Behälter.

Der Begriff "unmittelbar" ist dabei als Zeitraum von wenigen Sekunden bis eine Stunde, vorzugsweise bis 30 min, insbesondere bis 15 min zu verstehen.

Ein zweiter Gegenstand der vorliegenden Erfindung ist daher ein Verfahren zur Aufhellung und/oder Blondierung von menschlichen Haaren, welches dadurch gekennzeichnet ist, dass ein anwendungsbereites Mittel des ersten Erfindungsgegenstands auf menschliches Haar aufgetragen wird, bei einer Temperatur von Raumtemperatur bis 45 °C für eine Einwirkdauer von 10 bis 60 min verbleibt, und die Haare anschließend mit Wasser oder einer wässrigen, tensid-haltigen Zubereitung ausgespült werden.

Die erfindungsgemäßen Mittel werden in einem Verfahren zum Aufhellen von keratinischen Fasern, insbesondere menschlichen Haaren, angewendet, bei dem das Mittel des zweiten Erfindungsgegenstands auf die keratinhaltigen Fasern aufgebracht, 10 bis 60 Minuten auf der Faser belassen und anschließend mit Wasser wieder ausgespült oder mit einem Shampoo ausgewaschen wird.

Bevorzugt beträgt die Einwirkzeit der anwendungsbereiten Aufhellmittel 10 bis 60 min, insbesondere 15 bis 50 min, besonders bevorzugt 20 bis 45 min. Während der Einwirkzeit des Mittels auf der Faser kann es vorteilhaft sein, den Aufhellvorgang durch Wärmezufuhr zu unterstützen. Die Wärmezufuhr kann durch eine externe Wärmequelle, wie mit Hilfe eines Warmluftgebläses, als auch, insbesondere bei einer Haaraufhellung am lebenden Probanden, durch die Körpertemperatur des Probanden erfolgen. Bei letzterer Möglichkeit wird üblicherweise die aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Die Aufhellmittel ergeben bereits bei physiologisch verträglichen Temperaturen von unter 45°C gute Blondier- und Aufhellergebnisse.

Nach Ende der Einwirkzeit wird die verbleibende Aufhellzubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Leitungswasser erfolgen kann, wenn das Aufhellmittel einen stark tensid-haltigen Träger besitzt.

Die bevorzugten Ausführungsformen des ersten Erfindungsgegenstands gelten mutatis mutandis auch für den zweiten Erfindungsgegenstand.

Ein dritter Gegenstand der vorliegenden Erfindung ist die kosmetische Verwendung eines Mittels des ersten oder des zweiten Erfindungsgegenstands zur Verbesserung des Pflegezustands der Haare bei der Aufhellung und/oder Blondierung von menschlichen Haaren.

Die Ausführungsformen des ersten und zweiten Erfindungsgegenstands gelten mutatis mutandis für die Verwendung des dritten Erfindungsgegenstands.

Die Konfektionierung der erfindungsgemäßen Aufhellmittel unterliegt prinzipiell keinerlei Beschränkungen. Um dem Anwender die Komponenten des anwendungsbereiten Aufhellmittels des zweiten Erfindungsgegenstands möglichst komfortabel anzubieten, ist es sinnvoll, die einzelnen Zubereitungen in einer Verpackungseinheit gemeinsam zu vertreiben.

Ein vierter Gegenstand der vorliegenden Erfindung ist daher eine Mehrkomponentenverpackungseinheit (Kit-of-Parts) zur Aufhellung und/oder Blondierung von keratinischen Fasern, umfassend in jeweils getrennt voneinander konfektionierten Containern
a) mindestens ein pulverförmiges Mittel (A), enthaltend mindestens ein Peroxo-Salz,
b) mindestens einer Oxidationsmittelzubereitung (B), enthaltend in einem wässrigen, kosmetischen Träger mindestens Wasserstoffperoxid, und
c) mindestens einer Zubereitung (C), enthaltend in einem wässrigen kosmetischen Träger mindestens eine Ammonium-Verbindung der Formel (I) des ersten Erfindungsgegenstands.

Die Komponenten der Mehrverpackungseinheit werden getrennt voneinander in räumlich unterschiedlichen Behältern oder Containern konfektioniert. Der Begriff "Behälter" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff Behälter umfasst daher, ohne darauf beschränkt zu sein, das Innere einer Tube, eines Beutels oder Sackes, eines Kanisters, einer Dose, einer Wanne, einer Flasche, eines Glases oder eines Paketes, eines Kartons, einer Box, eines Umschlages oder eines anderen Behältnisses. Die Behälter können mit einer wiederverschließbaren Öffnung wie einem Schraubverschluss versehen sein. Dies kann insbesondere dann von Vorteil sein, wenn mehrere Mittel vor der Anwendung durch Schütteln innig miteinander vermischt werden sollten.

Die Komponenten der Aufhellzubereitung können in einem Doppelkammer-Behälter mit getrennter oder gemeinsamer Öffnung enthalten sein. Es ist jedoch bevorzugt, sie auf verschiedene Behälter aufzuteilen und den Verbraucher anzuleiten, sie vor der Anwendung miteinander zu mischen.

Bevorzugt enthält die Mehrkomponentenverpackungseinheit (Kit-of-Parts) zusätzlich eine Gebrauchsanleitung. Die Gebrauchsanleitung enthält insbesondere Informationen, Erläuterungen und gegebenenfalls Illustrationen für den Verbraucher (m/f) zur Anwendung der Mittel aus den Behältern der Verpackungseinheit in einem Verfahren gemäß dem zweiten Erfindungsgegenstand. Darüber hinaus kann es bevorzugt sein, wenn weiterhin eine Anmischhilfe, wie beispielsweise eine Schale, eine Applikationshilfe, wie beispielsweise ein Kamm oder ein Pinsel, und/oder eine persönliche Schutzausrüstung, wie beispielsweise Einweg-Handschuhe dem Kit beigefügt sind.

Bezüglich bevorzugter Ausführungsformen der erfindungsgemäßen Mehrkomponentenverpackungseinheit gelten mutatis mutandis die Ausführungsformen der vorangegangenen Erfindungsgegenstände.

### Beispiele

### 1.1 Blondiermittel

| **Booster (Komponente A)** | A1 | A2 | A3 | A4 | A5 |
|---|---|---|---|---|---|
| Kaliumpersulfat | 98,6 | - | 45 | 60 | 50 |
| Ammoniumpersulfat | - | 100 | 15 | 20 | - |
| Natriumpersulfat | - | - | 20 | 20 | 50 |
| Aerosil 200 | 1,4 | - | - | - | - |
| Britesil C20 | - | - | 20 | - | - |

| **Entwickler (Komponente B)** | B1 | B2 | B3 | B4 |
|---|---|---|---|---|
| Natronlauge 45 Gew.-% wässrig | 0,8 | 0,8 | 0,8 | 0,8 |
| Dipicolinsäure | 0,1 | 0,1 | 0,1 | 0,1 |
| Dinatriumpyrophosphat | 0,05 | 0,05 | 0,05 | 0,05 |
| HEDP, 60 Gew.-% wässrig | 1,5 | 1,5 | 1,5 | 1,5 |
| Natrium Laureth-2 sulfat, 27 Gew.-% wässrig | 2,0 | 2,0 | 2,0 | 2,0 |
| Dow Corning DB 110 A | 0,08 | 0,08 | 0,08 | 0,08 |
| Aculyn 33A | 15 | 15 | 15 | 15 |
| Wasserstoffperoxid, 50 % wässrig | 24 | 18 | 12 | 6 |
| Wasser, vollentsalzt | ad 100 | ad 100 | ad 100 | ad 100 |

| **Blondiercreme (Komponente C)** | C1 | C2 | C3 | C4 | C5 | C6 | C7 | C8 |
|---|---|---|---|---|---|---|---|---|
| Cetearylalkohol | 11,0 | 11,0 | 11,0 | 11,0 | 11,0 | 11,0 | 11,0 | 11,0 |
| Cocosfettalkohol | 2,75 | 2,75 | 2,75 | 2,75 | 2,75 | 2,75 | 2,75 | 2,75 |
| Ceteareth-20 | 0,25 | 0,25 | 0,25 | 0,25 | 0,25 | 0,25 | 0,25 | 0,25 |
| Varisoft W 575 PG | 3,0 | 5,0 | 4,0 | 4,0 | 5,0 | 5,0 | 2,0 | 3,0 |
| Natrium Laureth-2 sulfat, 27 Gew.-% wässrig | 26,5 | 26,5 | 26,5 | 26,5 | 26,5 | 26,5 | 26,5 | 26,5 |
| Ammoniumsulfat | 1,0 | - | 1,0 | - | 1,0 | - | 1,0 | 1,0 |
| HEDP, 60 Gew.-% wässrig | 0,2 | 0,2 | 0,2 | 0,2 | 0,2 | 0,2 | 0,2 | 0,2 |
| Natriumsilikat 40/42 | 0,5 | 0,5 | 0,5 | 0,5 | 0,5 | 0,5 | 0,5 | 0,5 |
| Sonnenblumenöl | 2,0 | 2,0 | 1,0 | 1,0 | - | - | - | 10 |
| Parfum | 0,1 | 0,1 | 0,1 | 0,1 | 0,1 | 0,1 | 0,4 | 0,4 |
| Monoethanolamin | 8,0 | 8,0 | 8,0 | 8,0 | 8,0 | 8,0 | - | - |
| Ammoniak, 25 Gew.-% wässrig | - | - | - | - | - | - | 7,6 | 7,6 |
| Wasser, vollentsalzt | ad 100 | | | | | | | |

Folgende Rohstoffe wurden eingesetzt:
Aerosil 200 (INCI-Bezeichnung: Silica; Evonik); Britesil C20 (INCI-Bezeichnung: Sodium Silicate;
PQ Europe); Dow Corning DB 110 A (ca. 10 Gew.-%; INCI-Bezeichnung: Aqua, Dimethicone; Dow Corning); Aculyn 33A (ca. 28 Gew.-%; INCI-Bezeichnung: Aqua, Acrylates Copolymer;
Rohm&Haas); Varisoft W 575 PG (ca. 75 Gew.-%; INCI-Bezeichnung: Quaternium-87, Propylene Glycol; Evonik).

### 1.2

Die Komponenten A:B:C werden üblicherweise in Verhältnis 1:6:6 zum anwendungsbereiten Blondiermittel vermischt. Dabei sind alle denkbaren Kombinationen zwischen A1 bis A5, B1 bis B4 und C1 bis C8 möglich.

### 2. Wirknachweise

### 2.1 Naßkämmarbeit

Zur Beurteilung der Naßkämmarbeit wurden jeweils 10 Haarsträhnen (Kerling 6/0) mit einem anwendungsbereiten Mittel M1 aus 1 Gewichtsteil A1, 6 Gewichtsteilen B1 und 6 Gewichtsteilen C1 sowie dem entsprechenden Mittel M2 ohne Ammoniumverbindung der Formel (I) für 45 Minuten bei 32 °C behandelt. Danach wurden die Haare gewaschen.

Vor der Anwendung der Testformulierungen wurden die wassernassen Strähnen 10x automatisch mit Hartgummikämmen (Hercules Sägemann) gekämmt und die aufzuwendende Arbeit bestimmt. Nach Anwendung der Mittel M1 und M2 wurden die Strähnen mit Wasser für 2 min gespült und die Messungen wiederholt. Es wurden jeweils 10 Haarsträhnen behandelt und gemessen. Als Wert für die Kämmarbeit wurde jeweils das arithmetische Mittel gebildet. Folgende Ergebnisse wurden erhalten:

| | Erhöhung der Naßkämmarbeit |
|---|---|
| unbehandelt | -- |
| M1 (mit Varisoft W 575 PG) | **+ 83%** |
| M2 (ohne Varisoft W 575 PG) | **+ 235 %** |

Die Ergebnisse zeigen eine deutliche Verbesserung der Naßkämmbarkeit durch den Zusatz der erfindungsgemäßen Ammoniumverbindung im pulverförmigen Blondiermittel.

### 2.2 Haarbruch und Haarspliss

Zur Beurteilung von Haarbruch und Spliss wurden jeweils 10 Haarsträhnen (Kerling 6/0) mit einem anwendungsbereiten Mittel M1 und M2 blondiert.

Die Strähnen wurden anschließend bei konstanter Temperatur und Luftfeuchtigkeit (25°C, 50 % rH) unter kontinuierlicher Entladung 20.000 mal mit einem feinzähnigen Hartgummi-Kamm gekämmt.
2.2.1 Zur Bestimmung von Spliss wurden nach Kämmvorgang eine 1,5 cm lange Spitze jeder Strähne abgeschnittem und in ein sehr feines Sieb (Mesh Größe 200 µm) überführt. Mit Hilfe eines Luftstroms von der Unterseite des Siebs wurden Haarspitzen mit Haarspliss von solchen ohne Haarspilss abgetrennt. Für jede Strähne wurde die Menge der Haare mit Spliss, die auf dem Sieb zurückbleiben, zu der Gesamtmenge der abgeschnitten Spitzen ins Verhältnis gesetzt.

| | Anteil an Haarspliss nach 20.000 Kämmwiederholungen |
|---|---|
| unbehandelt | -- |
| M1 (mit Varisoft W 575 PG) | **-29%** |
| M2 (ohne Varisoft W 575 PG) | **+33%** |

Es zeigte sich, dass der Anteil an Haarspliss für die mit nicht erfindungsgemäßen Mittel M2 behandelten Strähnen deutlich größer ist als bei unbehandelten Haaren und als bei mit erfindungsgemäßen Mittel M1 behandelten Strähnen. Die erfindungsgemäßen Mittel verringern den Anteil an Haarspliß gegenüber unbehandelten Strähnen sogar.
2.2.2 Zur Bestimmung von Haarbruch wurde während dem Kämmvorgang jeweils das abgebrochene Haar unter dem Kamm gesammelt. Die Menge des Haarbruchs wurde ausgewogen und zur Gesamtmenge der Strähne ins Verhältnis gesetzt.

| | Anteil an Haarbruch nach 20.000 Kämmwiederholungen |
|---|---|
| unbehandelt | -- |
| M1 (mit Varisoft W 575 PG) | **- 83 %** |
| M2 (ohne Varisoft W 575 PG) | **+15 %** |

Die Ergebnisse zeigen, dass der Anteil an Haarbruch für die mit nicht erfindungsgemäßen Mittel M2 behandelten Strähnen deutlich größer ist als bei unbehandelten Haaren und als bei mit erfindungsgemäßen Mittel M1 behandelten Strähnen. Die erfindungsgemäßen Mittel M1 verringern den Anteil an Haarbruch gegenüber unbehandelten Strähnen sehr deutlich.

### 2.3 Testsalon

Die Avivage zeigte nach Behandlung mit erfindungsgemäßen Mitteln M1 im nassen Haar ohne Conditioner bereits deutlich verbesserte Ergebnisse als im Haar, welches mit dem Mittel M2 behandelt worden war.

## Patentansprüche

1. Mittel zur Aufhellung und/oder Blondierung von keratinischen Fasern, welches unmittelbar vor der Anwendung auf der Faser aus mindestens 3 Teilen gemischt wird, und welches **dadurch gekennzeichnet ist, dass** die 3 Teile
i) eine wasserfreie Zubereitung (A), enthaltend mindestens ein Peroxo-Salz,
ii) eine wässrige Zubereitung (B), enthaltend mindestens Wasserstoffperoxid, und
iii) eine Zubereitung (C), enthaltend in einem wässrigen kosmetischen Träger mindestens eine Ammonium-Verbindung der Formel (I), 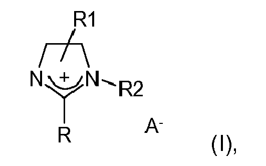 worin
R für eine gesättigte oder ungesättigte, lineare oder verzweigte C₇-C₂₉-Kohlenwasserstoffgruppe,
R1 an ein Stickstoffatom im Imidazolin-Ring gebunden ist und für eine C₁-C₆-Alkylgruppe, eine C₂-C₆-Alkenylgruppe oder eine C₂-C₆-Hydroxyalkylgruppe,
R2 für (CH₂)ₘXC(=O)R', worin m für eine ganze Zahl von 1 bis 5, X für O oder NH und R' für eine gesättigte oder ungesättigte, lineare oder verzweigte C₇-C₂₉Kohlenwasserstoffgruppe stehen, oder für (CH₂)ₙCO₂H mit n als ganzer Zahl von 1 bis 5, und
A für ein physiologisch verträgliches Anion stehen,
umfassen.

2. Mittel nach Anspruch 1, **dadurch gekennzeichnet, dass** es als Ammonium-Verbindung gemäß Formel (I) mindestens eine Verbindung enthält, in der R2 für (CH₂)ₘXC(=O)R',
worin m für eine ganze Zahl von 1 bis 5, X für NH und R' für eine gesättigte oder ungesättigte, lineare oder verzweigte C₇-C₂₉-Kohlenwasserstoffgruppe stehen,
steht und R1 für eine C₁-C₆-Alkylgruppe steht.

3. Mittel nach einem der Ansprüche 1 oder 2, **dadurch gekennzeichnet, dass** es als Ammonium-Verbindung gemäß Formel (I) mindestens eine Verbindung enthält, ausgewählt ist aus der Gruppe, die gebildet wird Quaternium-87 und Quaternium-27.

4. Mittel nach einem der Ansprüche 1 bis 3, **dadurch gekennzeichnet, dass** die Ammonium-Verbindungen der Formel (I) in einer Menge von 0,01 bis 20 Gew.-%, insbesondere von 0,5 bis 5 Gew.-% und besonders bevorzugt von 0,1 bis 3 Gew.-%, jeweils bezogen das Gesamtgewicht des Mittels, enthalten ist.

5. Mittel nach einem der Ansprüche 1 bis 4, **dadurch gekennzeichnet, dass** die Zubereitung (C) zusätzlich mindestens ein pflanzliches Öl, ausgewählt aus der Gruppe, die gebildet wird aus Sonnenblumenöl, Sojabohnenöl, Leinöl, Baumwollsaatöl, Rapsöl, Maiskeimöl, Weizenkeimöl und Erdnussöl, enthält.

6. Mittel nach einem der Ansprüche 1 bis 5, **dadurch gekennzeichnet, dass** es als Peroxo-Salz der Zubereitung (A) mindestens ein anorganisches Peroxodisulfat-Salz, insbesondere Ammoniumperoxodisulfat, Kaliumperoxodisulfat und/oder Natriumperoxodisulfat, enthält.

7. Mittel nach einem der Ansprüche 1 bis 6, **dadurch gekennzeichnet, dass** die Zubereitung (A) zusätzlich mindestens eine Silicium-haltige Verbindung, ausgewählt aus der Gruppe, die gebildet wird aus Kieselsäure, Alkalimetallsilicaten und Erdalkalimetallsilicaten.

8. Verfahren zur Aufhellung und/oder Blondierung von menschlichen Haaren, **dadurch gekennzeichnet, dass** ein anwendungsbereites Mittel nach einem der Ansprüche 1 bis 7 auf menschliches Haar aufgetragen wird, bei einer Temperatur von Raumtemperatur bis 45 °C für eine Einwirkdauer von 10 bis 60 min verbleibt, und die Haare anschließend mit Wasser oder einer wässrigen, tensid-haltigen Zubereitung ausgespült werden.

9. Kosmetische Verwendung eines Mittels nach einem der Ansprüche 1 bis 7 zur Verbesserung des Pflegezustands der Haare bei der Aufhellung und/oder Blondierung von menschlichen Haaren.

10. Mehrkomponentenverpackungseinheit (Kit-of-Parts) zur Aufhellung und/oder Blondierung von keratinischen Fasern, umfassend in jeweils getrennt voneinander konfektionierten Containern
(i) mindestens ein pulverförmiges Mittel (A), enthaltend mindestens ein Peroxo-Salz,
(ii) mindestens einer Oxidationsmittelzubereitung (B), enthaltend in einem wässrigen, kosmetischen Träger mindestens Wasserstoffperoxid, und
(iii) mindestens einer Zubereitung (C), enthaltend in einem wässrigen kosmetischen Träger mindestens eine Ammonium-Verbindung der Formel (I) nach Anspruch 1.
